(19) Europäisches Patentamt / European Patent Office / Office européen des brevets

(11) **EP 3 812 763 B1**

(12) **EUROPEAN PATENT SPECIFICATION**

(45) Date of publication and mention of the grant of the patent:
**03.05.2023 Bulletin 2023/18**

(21) Application number: **20163643.8**

(22) Date of filing: **17.03.2020**

(51) International Patent Classification (IPC):
**G01N 33/38** (2006.01)    **G01N 5/04** (2006.01)

(52) Cooperative Patent Classification (CPC):
**G01N 33/38; G01N 5/04; Y02W 30/91**

(54) **METHOD OF DETERMINING THE CONTENT OF POLYMER MICROFIBRES IN CEMENT COMPOSITES BASED ON UTILISATION OF THE BUOYANCY PHENOMENON**

VERFAHREN ZUR BESTIMMUNG DES GEHALTES AN POLYMEREN MIKROFASERN IN ZEMENTZUSAMMENSETZUNGEN UNTER AUSNUTZUNG DES AUFTRIEBSPHÄNOMENS

MÉTHODE DE DÉTERMINATION DE LA TENEUR EN MICROFIBRES DE POLYMÈRES DANS LES COMPOSITIONS DE CIMENT BASÉE SUR L'UTILISATION DU PHÉNOMÈNE DE FLOTTABILITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.10.2019 PL 43157419**

(43) Date of publication of application:
**28.04.2021 Bulletin 2021/17**

(73) Proprietor: **Instytut Techniki Budowlanej 00-611 Warszawa (PL)**

(72) Inventor: **Chylinski, Filip 05-092 Dziekanow Lesny (PL)**

(74) Representative: **AOMB Polska Sp. z.o.o. ul. Rondo Ignacego Daszynskiego 1 00-843 Warsaw (PL)**

(56) References cited:
**CN-B- 105 606 479    CN-B- 105 806 737 JP-A- 2006 275 644**

**Description**

The invention relates to a method of determining content of polymer microfibres in cement composites, in particular in concrete and mortar.

Polymer fibres added to cement composites, according to PN-EN 14889-2 are classified as microfibres - class 1 with diameter less than 0.30 mm and macrofibres - class 2 with diameter greater than 0.30 mm. The macrofibres are added to concretes and mortars to increase their tensile strength, while the role of microfibres is to reduce the shrinkage cracks. The additional classification of microfibres in the above standard relates to classes Ia and Ib, i.e., single and fibrillated ones, respectively.

The use of fibres for concrete on an increasing scale sometimes forces to perform checks of how much microfibres have been actually dosed. Macrofibres over 0.30 mm in diameter, steel or polymer ones, can be separated from the concrete mix by washing on a sieve or from hardened concrete after it has been crushed, in compliance with PN-EN 14488-7:2007 "Testing sprayed concrete. Part 7: Fibre content of fibre reinforced concrete.".

The application of the above method to polymer fibres with much smaller diameters, less than 0.30 mm, and mostly shorter lengths, is impossible. A literature survey did not show publications on testing methods allowing for determining content of microfibres in mortars or concrete. At present, there are no standardized testing methods allowing for determining the content of polymer microreinforcement dispersed in cement composites.

CN 105606479 reveals a method for detecting a volume fraction of propylene fiber content in concrete. The method includes: selecting a sample and measuring the sample volume, crushing and grinding the sample, conducting rinsing and water injection and performing stirring. After one stirring cycle it is observed whether all polypropylene fibres suspend on the surface of the solution. Stirring is conducted until all the polypropylene fibres suspend on the solution surface, then the fibres are dried in the sun and weighted, thus obtaining the polypropylene fibre volume ratio.

CN105806737 reveals a method for determining polypropylene fibre content of building mortar. The method includes: preparing a sample, adding distilled water and stirring, aging the sample for 20-28 h at 13-18 °C, separating, extracting using tetrahydrofuran solution, neutralizing, filtering, drying and calculating the fibre content. The method of CN105806737 is not based on utilisation of buoyancy phenomenon - instead the microfibres are dissolved by THF.

JP2006275644 reveals a method for deriving the fibre content in a pulverized product of an inorganic molded product. The method is based on stepwise removal of water and thermal treatment of the polymer at 450-500°C.

A method of determining content of polymer microfibres in cement composites based on utilisation of the buoyancy phenomenon, according to the invention wherein a cement composite sample containing microfibres is crushed to grains less than 1 mm in size, the crushed mixture is flooded with a liquid with a density greater than that of the microfibres, but less than the density of a cement matrix, and after mixing and sedimentation the microfibres are collected from the surface, characterised in that after collection of microfibres a portion of an inorganic acid (including but not limited to, e.g., hydrochloric, nitric, sulphuric, phosphoric acid) is added to the liquid to set free any microfibres bound to the cement matrix, and after mixing, additional microfibres are collected from the surface, wherein the addition of acid and collection of microfibres operations are repeated until no microfibres come up to the surface, and then the microfibres thus separated are dried and weighed, and in case they are contaminated, they are additionally ignited at the polypropylene decomposition temperature, as a result of which the microfibres will decompose and only impurities remain whose weight after weighing is subtracted from the dried microfibres, and the final result obtained will indicate the content of microfibres in the tested sample.

Preferably, water is used as a liquid to flood the crushed composite sample.

Preferably, hydrochloric acid is used as an inorganic acid.

Preferably, the dried contaminated microfibres are ignited at $500\pm30$°C.

The solution according to the invention allows for accurate determination of the content of microfibres in the tested sample, and thus in the concrete or mortar used.

The method according to the invention is explained in detail in the following embodiment.

A minimum of 500 g of hardened and dried mortar or not less than 1 dm$^3$ concrete should be taken for testing. Then, the collected sample should be crushed and ground to grains of about 0.5 mm and smaller in size. Weigh approximately 500 g of the sample with an accuracy of 0.01 g ($m_S$) and place in a glass beaker with a capacity of about 3 litres. Flood with about 1.5 litres of water. Mix gently the whole using a glass rod or other resistant to alkali and acids. After the content has slightly stabilised, using a plastic or glass spoon gently collect the floating microfibres from the surface and transfer quantitatively to a 250 ml beaker. While stirring, add 50 ml concentrated hydrochloric acid to the solution. After the turbulent reaction has stopped, stop stirring and leave for about 2 minutes. Collect another portion of microfibres from the surface into a previously prepared beaker. Repeat the acid addition and fibre collection operation three more times. If addition of the last acid portion does not result in fibres further coming up to the surface, dispensing of the acid can be stopped.

The contents of the beaker with the collected microfibres should be filtered under vacuum through a prepared filter. Wash the filter with distilled water. Transfer the filter with its contents into a Petri dish and dry at 80°C for a minimum of 4 hours. After cooling down in a desiccator, weigh the whole on an analytical balance with an accuracy of 0.0001 g ($m_1$).

Ignite empty quartz or porcelain crucibles at 500 ± 30°C for 1 hour, then cool them down in a desiccator and weigh. Determine the residue after igniting at 500°C only the filter that was used for filtration of the microfibres, with an accuracy of 0.0001 g ($m_2$).

Transfer the previously dried filter with microfibres quantitatively to an empty, ignited and weighed crucible and weigh with an accuracy of 0.0001 g ($m_3$). Place the crucible with a ceramic lid into an furnace at 500 ± 30°C for 2 hours. After 30 minutes, remove the lid from the crucible and continue heating the crucible without cover. After cooling the crucible down in the desiccator, weigh the crucible with an accuracy of 0.0001 g ($m_4$).

Calculate the content of microfibres in mortar/concrete with the following formula:

$$M = \frac{(m_1 - m_0) - (m_4 - m_2 - m_3)}{m_S} \times 1000$$

wherein:

M - content of microfibres in mortar/concrete [g/kg],
$m_0$ - weight of Petri dish with dried filter [g],
$m_1$ - weight of Petri dish with dried microfibres and filter [g],
$m_2$ - residue after roasting the filters themselves at 500°C [g],
$m_3$ - weight of empty crucible [g],
$m_4$ - weight of crucible with filter and microfibres after igniting [g],
$m_S$ - weight of mortar/concrete sample used for testing [g].

If the content of microfibres is to be given per unit volume, as is often the case for concrete (kg/m$^3$), the density of the hardened mortar/concrete should be determined in advance. Then the content of microfibres is determined with the formula:

$$M_V = \frac{M \times d}{1000}$$

wherein:

$M_V$ - content of microfibres in mortar/concrete [kg/m$^3$],
d - density of mortar/concrete [kg/m$^3$].

**Example of performed mortar test**

A mortar sample weighing about 2 kg was dried at 80°C and crushed to grains smaller than 1 mm in size. 527.47 g of crushed mortar ($m_s$) was taken and transferred to a 3 dm$^3$ beaker. About 1.5 dm$^3$ of water was added and mixed. After the suspension had partially settled (about 2 minutes), the fibres were collected from the surface to another empty 250 ml beaker. 50 ml concentrated hydrochloric acid was added to the mixture. The whole was mixed using a glass rod. After partial settling of the suspension, the surface-floating fibres were collected into the previous beaker. The acid addition operations were repeated three more times. Adding the last portion of acid and mixing did not result in further fibres coming up to the surface.

Using a vacuum filtration set, an empty filter was prepared and washed with demineralised water. The filter was then dried in a Petri dish at 80°C. After cooling down in a desiccator, the dish with filter was weighed ($m_0$ = 59.0087 g). The collected fibres were filtered through a filter thus prepared and washed with demineralised water. The filter with fibres was then transferred to the same Petri dish and dried at 80°C. After cooling down in the desiccator, the whole was weighed ($m_1$ = 59.8963 g). The weight of contaminated fibres was: 0.8876 g.

Loss on ignition of the filter at 500°C was determined ($m_2$ = 0.0015 g). The empty crucible was ignited and weighed after cooling ($m_3$ = 17.5403 g). The filter with fibres was placed in it. The whole was ignited at 500°C for 2 hours (for the first 30 minutes under cover). After cooling down in a desiccator, the crucible was weighed ($m_4$ = 18.1169 g). The weight of impurities remaining after ignition was: 0.5766 g.

The content of fibres in the tested sample was: 0.8876 - 0.5766 = 0.3110 g.

The content of fibres in the tested mortar was: 0.59 g/kg.

The volume content of fibres in the tested mortar after prior determination of the density of the tested mortar

(2060.43 kg/m$^3$) was 1,21 kg/m$^3$.

**Claims**

1. Method of determining content of polymer microfibres in cement composites based on utilisation of the buoyancy phenomenon, wherein a cement composite sample containing microfibres is crushed to grains less than 1 mm in size, the crushed mixture is flooded with a liquid with a density greater than that of the microfibres, but less than the density of a cement matrix, and after mixing and sedimentation the microfibres are collected from the surface, **characterised in that** after collection of microfibres a portion of an inorganic acid is added to the liquid to set free any microfibres bound to the cement matrix, and after mixing additional microfibres are collected from the surface, wherein the addition of acid and collection of microfibres operations are repeated until no further microfibres come up to the surface, and then the microfibres thus separated are dried and weighed, and in case they are contaminated, they are additionally ignited at the polypropylene decomposition temperature, as a result of which the microfibres will decompose and only impurities remain whose weight after weighing is subtracted from the dried microfibres, and the final result obtained will indicate the content of microfibres in the tested sample.

2. Method according to claim 1 **characterised in that** water is used as a liquid to flood the crushed composite sample.

3. Method according to claim 1 **characterised in that** hydrochloric acid is used as an inorganic acid.

4. Method according to claim 1 **characterised in that** dried contaminated microfibres are ignited at 500±30°C.


**Patentansprüche**

1. Ein Verfahren zur Bestimmung des Gehalts an polymeren Mikrofasern in Zementverbundwerkstoffen unter Verwendung des Auftriebsphänomens, wobei eine Mikrofasern enthaltende Zementverbundwerkstoffprobe zu Körnern mit einer Größe von weniger als 1 mm zerkleinert wird, das zerkleinerte Gemisch mit einer Flüssigkeit geflutet wird, deren Dichte größer als die der Mikrofasern, aber kleiner als die Dichte einer Zementmatrix ist, und nach dem Mischen und Sedimentieren die Mikrofasern von der Oberfläche gesammelt werden, **dadurch gekennzeichnet, dass** nach dem Sammeln der Mikrofasern ein Teil einer anorganischen Säure zu der Flüssigkeit hinzugefügt wird, um jegliche an die Zementmatrix gebundenen Mikrofasern freizusetzen, und nach dem Mischen weitere Mikrofasern von der Oberfläche gesammelt werden, wobei die Vorgänge der Zugabe von Säure und des Sammelns von Mikrofasern so lange wiederholt werden, bis keine weiteren Mikrofasern mehr an die Oberfläche kommen, und dann die so abgetrennten Mikrofasern getrocknet und gewogen werden, und falls sie kontaminiert sind, werden sie zusätzlich bei der Zersetzungstemperatur von Polypropylen entzündet, wodurch sich die Mikrofasern zersetzen und nur Verunreinigungen übrig bleiben, deren Gewicht nach dem Wiegen von den getrockneten Mikrofasern abgezogen wird, und das so erhaltene Endergebnis gibt den Gehalt an Mikrofasern in der untersuchten Probe an.

2. Das Verfahren nach Anspruch 1, **dadurch gekennzeichnet, dass** Wasser als Flüssigkeit zum Fluten der zerkleinerten Mischprobe verwendet wird.

3. Das Verfahren nach Anspruch 1, **dadurch gekennzeichnet, dass** als anorganische Säure Salzsäure verwendet wird.

4. Das Verfahren nach Anspruch 1, **dadurch gekennzeichnet, dass** getrocknete kontaminierte Mikrofasern bei 500±30°C entzündet werden.


**Revendications**

1. Méthode de détermination de la teneur en microfibres de polymère dans les composites de ciment basée sur l'utilisation du phénomène de flottabilité, dans laquelle un échantillon de composite de ciment contenant des microfibres est broyé en grains d'une taille inférieure à 1 mm, le mélange broyé est inondé d'un liquide dont la densité est supérieure à celle des microfibres, mais inférieure à la densité d'une matrice de ciment, et après mélange et sédimentation, les microfibres sont collectées à la surface, **caractérisé en ce qu'**après la collecte des microfibres, une partie d'un acide inorganique est ajoutée au liquide pour libérer toutes les microfibres liées à la matrice de ciment, et qu'après le mélange, des microfibres supplémentaires sont collectées à la surface, les opérations d'ajout d'acide et de collecte des microfibres étant répétées jusqu'à ce que plus aucune microfibre ne remonte à la surface, puis les microfibres ainsi séparées sont séchées et pesées et, au cas où elles seraient contaminées, elles sont en outre enflammées à la température de décomposition du polypropylène, ce qui a pour effet de décomposer les microfibres et de ne laisser subsister que les impuretés dont le poids après pesée est soustrait des microfibres séchées, et le résultat final obtenu indique la teneur en microfibres de l'échantillon testé.

2. Méthode selon la revendication 1, **caractérisé en ce que** de l'eau est utilisée comme liquide pour inonder l'échantillon composite broyé.

3. Méthode selon la revendication 1, **caractérisé en ce que** l'acide chlorhydrique est utilisé comme acide inorganique.

4. Méthode selon la revendication 1, **caractérisé en ce que** les microfibres contaminées séchées enflammées à $500\pm30°C$.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 105606479
- CN 105806737

- JP 2006275644 B